# EUROPEAN PATENT SPECIFICATION

(11) **EP 0 781 268 B1**
(45) Date of publication and mention of the grant of the patent: **07.04.1999**
(21) Application number: 95932664.6
(22) Date of filing: 05.09.1995
(51) Int. Cl.: C07C 215/44, A61K 31/135

(54) **DERIVATIVES OF 2-AMINO-1,2,3,4-TETRAHYDRONAPHTHALENE ACTIVE ON THE CARDIOVASCULAR SYSTEM**
KARDIOVASKULÄR AKTIVE 2-AMINO-1,2,3,4-TETRAHYDRONAPHTHALENE
DERIVES DE 2-AMINO-1,2,3,4-TETRAHYDRONAPHTALENE AGISSANT SUR LE SYSTEME CARDIO-VASCULAIRE

(30) Priority: 13.09.1994 IT MI941869
(43) Date of publication of application: 02.07.1997
(73) Proprietor: ZAMBON GROUP S.p.A., I-36100 Vicenza (IT)
(72) Inventor: MONTANARI, Stefania, I-20139 Milano (IT); BOTTA, Daniela, I-22100 Como (IT); CAVALLERI, Paolo, I-20144 Milano (IT); SANTANGELO, Francesco, I-20148 Milano (IT)
(86) International application number: EP9503479
(87) International publication number: WO9608463

(56) References cited:
- EP-A- 0 072 061
- WO-A-93/19036
- WO-A-95/07885

## Description

The present invention relates to compounds active on the cardiovascular system and, in particular, it relates to derivatives of 2-amino-1,2,3,4-tetrahydro-naphthalene and to their use in therapeutic field.

European patent application no. 72061 (Fisons PLC) describes, among the others, dopamines and aminotetrahydronaphthalenes having a mono-or di-substituted portion of formula 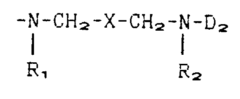 wherein
X is a -(CH₂)ₙ- chain, optionally substituted by hydroxy; n is an integer between 1 and 7; R₁ and R₂, the same or difference, are hydrogen, alkyl or phenyl; D₂ is hydrogen, alkyl, phenyl; alkyl substituted by one or more hydroxy, pyridyl, phenyl; alkyl substituted by phenyl substituted, in turn, by halogen, alkyl, amino, alkoxy or nitro; or D₂ may be a group of formula 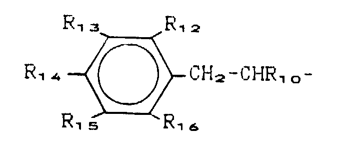 wherein none, one or two among R₁₃, R₁₄, R₁₅ and R₁₆ are hydroxy, the remaining being hydrogen; R₁₂ and R₁₀ are hydrogen atoms or together can form a -CH₂-CH₂- chain.

Among the compounds described in European patent application no. 72061, the compound of formula 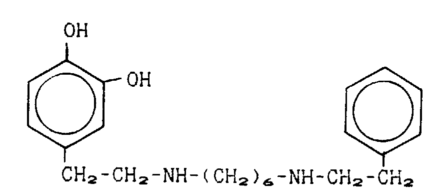 whose International non-proprietary name is dopexamine (The Merck Index - XI ed., No. 3418, page 538) is the only compound, as far as we know, which has been developed and used in the acute treatment of failure.

It is significant that dopexamine, notwithstanding it was selected among the several compounds described and exemplified in European patent application no. 72061, is an agonist of dopaminergic receptors less active than dopamine and, like dopamine itself, it is not absorbed when administered by oral route [A. Fitton and P. Benfield, Drugs, 39(2), 308-330, (1990)].

The patent application WO 95/07885 (in the name of the applicant) which falls under the provisos of Article 54(3), discloses 2-amino-1,2,3,4-tetrahydronaphthalene derivatives active on the cardiovascular system through a dopaminergic mechanisms of action.

Also the patent application WO 93/19036 (in the name of tie applicant) describes 2-amino-1,2,3,4-tetrahydronaphthalene derivatives endowed with dopaminergic activity.

The present invention is based on the finding that compounds of formula 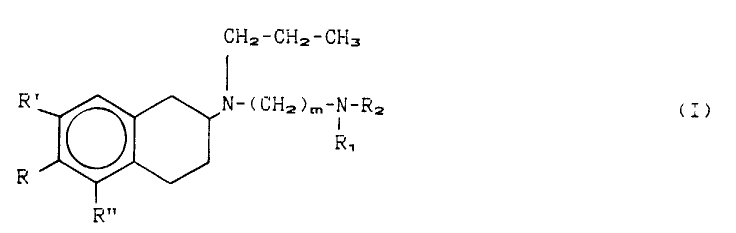 wherein
- m: is an integer number selected among 4, 5, 6, 7 and 8;
- R, R' and R": are hydrogen atoms or OH groups, provided that at least one among R, R' and R" is a hydrogen atom but R, R' and R" are not all contemporaneously hydrogen atoms and R' and R" are not both contemporaneously OH groups;
- R₁: is a hydrogen atom or a C₁-C₄ alkyl group;
- R₂: is a -(CH₂)ₙ-Ar group wherein
n is an integer number selected among 2, 3, 4 and 5;
Ar is a phenyl or
- R₂: is a group of formula 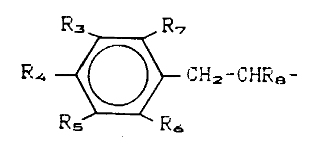 wherein one or two among R₃, R₄, R₅ and R₆ are OH groups and the remaining are hydrogen atoms;
R₇ and R₈ are hydrogen atoms or together can form a -CH₂-CH₂-chain;

which are comprised but not exemplified in the above European patent application No. 72061 are endowed with a better pharmacological activity and with improved pharmacokinetic characteristics in comparison with dopexamine.

Therefore, object of the present invention are the compounds of formula 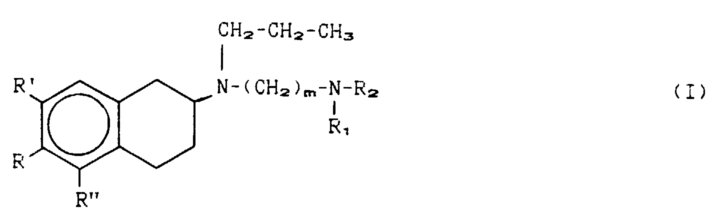 wherein
- m: is an integer number selected among 4, 5, 6, 7 and 8;
- R, R' and R": are hydrogen atoms or OH groups, provided that at least one among R, R' and R" is a hydrogen atom but R, R' and R" are not all contemporaneously hydrogen atoms and R' and R" are not both contemporaneously OH groups;
- R₁: is a hydrogen atom or a C₁-C₄ alkyl group;
- R₂: is a -(CH₂)ₙ-Ar group wherein
n is an integer number selected among 2, 3, 4 and 5;
Ar is a phenyl or
- R₂: is a group of formula 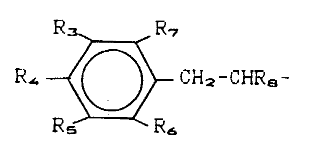 wherein one or two among R₃, R₄, R₅ and R₆ are OH groups and the remaining are hydrogen atoms;
R₇ and R₈ are hydrogen atoms or together can form a -CH₂-CH₂-chain;
and pharmaceutically acceptable salts thereof.

The compounds of formula I have at least an asymmetric center and they may be in the form of stereoisomers.

Object of the present invention are the compounds of formula I in the form of stereoisomeric mixtures as well as in the form of single stereoisomers.

The compounds of formula I are agonists of dopaminergic receptors active also by oral route and with long duration of action and they are useful in therapy in cardiovascular field, in particular for the treatment of arterial hypertension and heart failure, of renal failure, in the treatment of peripheral arteriopathies, of cerebrovascular insufficiencies and of ischemic cardiopathy.

Preferred compounds of formula I are the compounds wherein R₂ is a group of formula -(CH₂)ₙ-Ar wherein Ar is a phenyl and n is 2 or is a group of formula 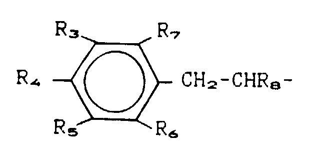 wherein one or two among R₃, R₄, R₅ and R₆ are OH groups and the remaining are hydrogen atoms; R₇ and R₈ are hydrogen atoms. Still more preferred compounds are the compounds wherein m is 6; R₂ is a group of formula -(CH₂)ₙ-Ar wherein Ar is a phenyl and n is 2 or is a group of formula 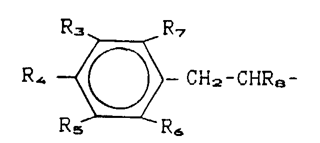 wherein one or two among R₃, R₄, R₅ and R₆ are OH groups and the remaining are hydrogen atoms; R₇ and R₈ are hydrogen atoms. Pharmaceutically acceptable salts of the compounds of formula I are the salts with organic and inorganic acids such as, for example, hydrochloric, hydrobromic, hydroiodic, nitric, sulphuric, phosphoric, acetic, benzoic, maleic, fumaric, succinic, tartaric, citric, aspartic, methansulphonic and 3,7-di-tert-butylnaphthalen-1,5-disulphonic acid (dibudinic acid).

The preparation of the compounds of formula I can be carried out according to the synthetic method described herein after.

The method comprises the reaction between a compound of formula 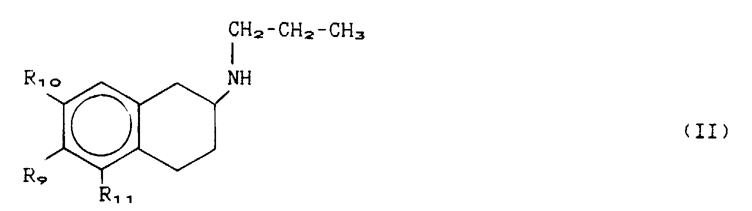 wherein
- R₉, R₁₀ and R₁₁: are hydrogen atoms or OY groups wherein Y is a hydrogen atom or a protective group selected, for example, among methyl, benzyl, benzoyl and 4-methoxybenzoyl, provided that at least one among R₉, R₁₀ and R₁₁ is a hydrogen atom but R₉, R₁₀ and R₁₁ are not all contemporaneously hydrogen atoms and R₁₀ and R₁₁ are not both contemporaneously OY groups;
and an acid of formula 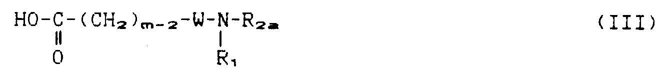 wherein m and R₁ have the already reported meanings; W is a CH₂ or CO group; R₂ₐ has the meanings already reported for R₂ or is a group of formula 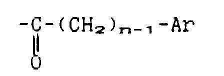 or of formula 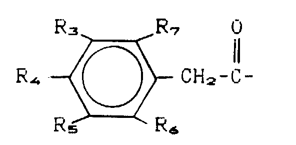 wherein n Ar, R₃, R₄, R₅ and R₆ have the already reported meanings; R₇ is a hydrogen atom;
or a reactive derivative thereof such as an acyl halide or a mixed anhydride which can optionally be prepared in situ, in an inert solvent and in the presence of a base such as an alkaline carbonate or bicarbonate or a tertiary amine,
in order to obtain the intermediate compounds of formula 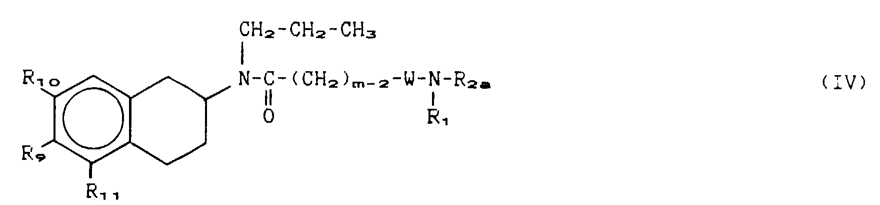 wherein m, W, R₁, R₂ₐ, R₉, R₁₀ and R₁₁ have the already reported meanings;
and their reduction, preceded or followed by the optional deprotection of the hydroxy groups, in order to obtain the compounds of formula I.

The reduction of the compounds of formula IV can be carried out with electrophile reducing agents, in particular with diborane optionally complexed with dimethylsulphide, tetrahydrofuran, aliphatic amines such as triethylamine or aromatic amines such as N,N-diethylaniline or pyridine.

Alternatively, the reduction can be carried out with nucleophile reducing agents such as metal hydrides, for example lithium aluminum hydride.

The reduction reaction is carried out in a suitable solvent such as for example tetrahydrofuran, diethylether or 1,2-dimethoxyethane. The optional deprotection of the hydroxy groups is carried out according to conventional techniques such as hydrolysis or hydrogenolysis.

The compounds of formula II are known or easily prepared according to known methods (British patent No. 1509454 - The Wellcome Foundation Ltd.).

Also the compounds of formula III are known or easily prepared according to conventional methods.

For example the compounds of formula III wherein W is a CH₂ group can be prepared by condensation between an amino acid of formula 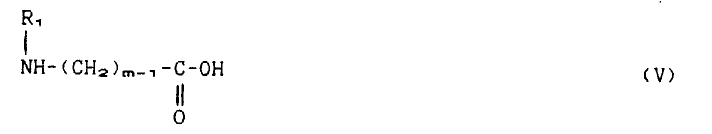 wherein m and R₁ have the already reported meanings; and a halide of formula

X-Z-(CH₂)ₙ₋₁-Ar (VI)

or of formula 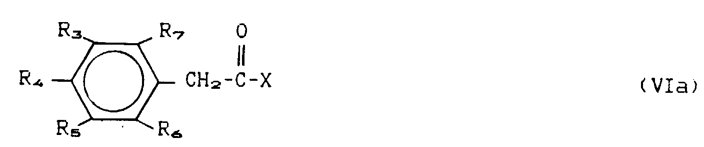 or of formula 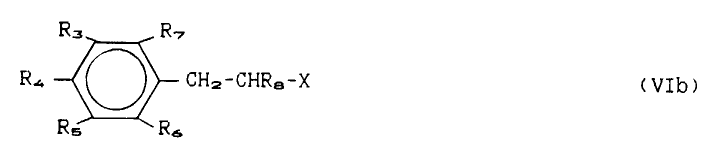 wherein n Ar, R₃, R₄, R₅, R₆, R₇ and R₈ have the already reported meanings; X is a chlorine or bromine atom and Z is a CH₂ or CO group.

The compounds of formula III wherein W is a CO group and R₂ₐ has the same meanings of R₂ can be prepared by condensation between an amine of formula 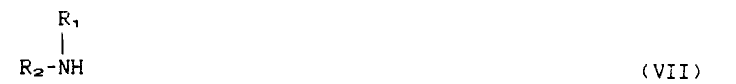 wherein R₁ and R₂ have the already reported meanings;
and a bicarboxylic acid of formula 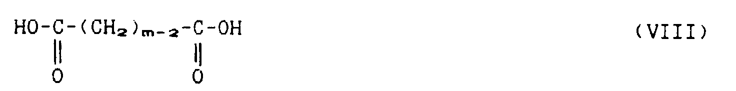 wherein m has the already reported meanings, suitably activated.

The intermediates of formula V, VI, VIa, VIb, VII and VIII are commercially available (Aldrich) or easily prepared by known methods starting from commercially available compounds.

Alternatively, the synthesis of the compounds of formula I can be carried out following a different sequence.

Therefore, the compounds of formula II can be reacted, at first, with an amino acid of formula V or a reactive derivative thereof in order to obtain the intermediate of formula 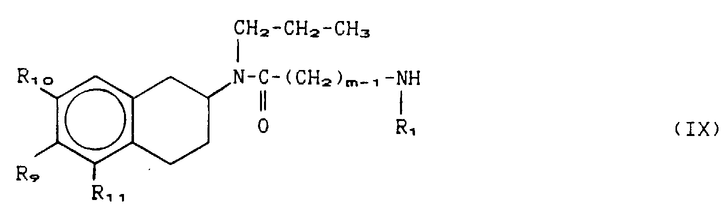 wherein m, R₁, R₉, R₁₀ and R₁₁ have the already reported meanings;
which is then reacted with a halide of formula VI, VIa or VIb obtaining the intermediates of formula 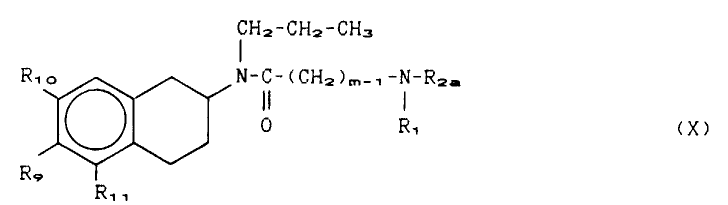 wherein m, R₁, R₂ₐ, R₉, R₁₀ and R₁₁ have the already reported meanings;

The subsequent reduction, preceded or followed by the optional deprotection of the hydroxy groups, gives the compounds of formula I, object of the present invention.

Alternatively, the compounds of formula I can be prepared following a synthetic method which consists in first reducing a compound of formula IX, according to the already described reduction methods, and then reacting the resultant intermediate of formula 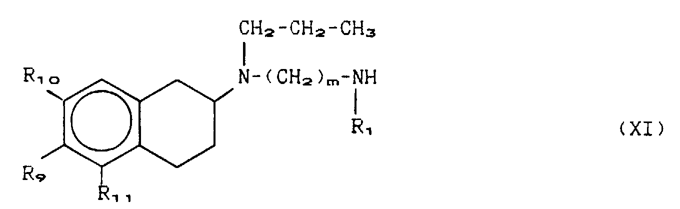 wherein m, R₁, R₉, R₁₀ and R₁₁ have the already reported meanings;
with a halide of formula VI, VIa or VIb obtaining the corresponding compounds of formula I after optional reduction and optional deprotection.

The compounds of formula I in optically active form are obtained by optical separation or by stereospecific or stereoselective synthesis.

The preparation of salts of the compounds of formula I is carried out according to conventional methods.

The compounds of formula I are agonists of D₁ and D₂ dopaminergic receptors having more affinity than dopamine and than dopexamine as results from the in vitro binding tests (example 21).

The in vitro results have been confirmed also by functional studies on isolated tissues, which are predictive of the in vivo activity, such as the Rabbit Splenic Artery (RSA) test and the Rabbit Ear Artery (REA) test (example 22).

The tests for interaction with the other receptor systems showed that the compounds of formula I do not significantly interact and then are endowed with high specificity.

The compounds of formula I resulted also to be inactive on the central nervous system after oral administration and this lack of effect is a further positive property generally not shared with the other compounds with cathecolamine structure.

It is clear how these characteristics of selectivity and receptor specificity together with the lack of activity on the central nervous system make the compounds of formula I particularly suitable for the treatment of cardiovascular diseases and mainly in the antihypertensive therapy, in the therapy of heart failure, of renal insufficiency, in the treatment of peripheral arteropathies, of cerebrovascular insufficiencies and of ischemic cardiopathy. In addition to the already underlined higher pharmacologic activity, the feature which more characterizes the compounds of formula I, object of the invention is their absorbability by oral route and their prolonged duration of action.

As a consequence for the practical uses in therapy, the compounds of formula I can be administered by parenteral route as well as by enteral route so differing from dopamine and from dopexamine.

The therapeutic doses will be generally comprised between 5 mg and 1 g a day and between 1 and 300 mg by oral route for each single administration.

The pharmaceutical compositions containing a therapeutically effective amount of the compounds of formula I or of their pharmaceutically acceptable salts in admixture with a suitable carrier are, furthermore, object of the present invention.

The pharmaceutical compositions object of the invention may be liquid, suitable for enteral or parenteral administration, and, preferably, solid such as tablets, capsules, granulates, suitable for oral administration.

The preparation of the pharmaceutical compositions object of the invention can be carried out according to traditional techniques.

Notwithstanding the compounds of formula I are active as such also when orally administered, in order to fulfill some specific therapeutic or pharmaceutical requirements it can be useful to transform them into the corresponding pro-drugs.

According to the techniques used in the field of phenolic and cathecolic derivatives, suitable pro-drugs are obtained by esterification of one or two hydroxy groups with pharmaceutically acceptable salts.

Specific examples of pro-drugs of the compounds of formula I are acetoxy derivatives, wherein the hydroxy groups are esterified with acetic acid, and mono- or di-phosphonates, wherein one or both hydroxy groups are esterified with phosphoric acid.

The compounds of formula I also when transformed into pro-drugs and in particular the compounds obtained by esterification of the phenolic hydroxy groups or of one or both the cathecolic hydroxy groups with pharmaceutically acceptable acids, as well as the pharmaceutical compositions which contain a compound of formula I in the form of a corresponding pro-drug, and in particular which contain a compound of formula I wherein the phenolic hydroxy groups or of one or both the cathecolic hydroxy groups are esterified with pharmaceutically acceptable acids are within the scope of the present invention.

In order to better illustrate the present invention the following examples are now given.

The chromatographic purifications were carried out on silica gel (230-400 mesh) columns.

The mass spectra were carried out under the following conditions: chemical ionization, isobutane, positive ions.

### Example 1

### Preparation of (S)-N-propionyl-5,6-dimethoxy-1,2,3,4-tetrahydro-2-naphthylamine

Propionyl chloride (14.3 mi; 165 mmoles) was added to a solution of (S)-5,6-dimethoxy-1,2,3,4-tetrahydro-2-naphthylamine (31 g; 150 2 mmoles) and triethylamine (23 ml; 165 mmoles) in N,N-dimethylformamide (310 ml) at room temperature under nitrogen.

The reaction mixture was kept under stirring for 1 hour, then poured into water (1.5 1) and the resultant solid was filtered by washing with water.

After drying at 50°C under vacuum, (S)-N-propionyl-5,6-dimethoxy-1,2,3,4-tetrahydro-2-naphthylamine (32.8 g) was obtained.
m.p. 149-151°C

¹H-NMR (300 MHz, CDCl₃): δ (ppm): 1.14 (t, 3H); 1.70-1.80 (m, 1H); 2.02 (m, 1H); 2.18 (q, 2H); 2.57 (dd, 1H); 2.75-3.00 (m, 2H); 3.04 (dd, 1H); 3.80 (s, 3H); 3.84 (s, 3H); 4.25 (m, 1H); 5.47 (bd, 1H); 6.74 (d, 1H); 6.78 (d, 1H).
Mass: 264 [M+1], 190.

### Example 2

### Preparation of (S)-N-propyl-5,6-dimethoxy-1,2,3,4-tetrahydro-2-naphthylamine hydrochloride

Borane-dimethylsulphide complex (82 ml; 854.4 mmoles) was added dropwise to a solution of (S)-N-propionyl-5,6-dimethoxy-1,2,3,4-tetrahydro-2-naphthylamine (22.5 g; 85.4 mmoles), prepared as described in example 1, in anhydrous tetrahydrofuran (900 ml), at room temperature and under nitrogen.

The reaction mixture was heated under reflux for 1.5 hours.

After cooling at 15°C, a solution of hydrochloric acid at 36% (9.5 ml) in methanol (247 ml) was cautiously added dropwise.

The reaction mixture was heated under reflux for 1 hour, then the solvent (about 500 ml) was distilled at atmospheric pressure and evaporated to dryness under vacuum.

The crude residue was collected with absolute ethanol and the solution was heated under reflux obtaining, after cooling and filtering, (S)-N-propyl-5,6-dimethoxy-1,2,3,4-tetrahydro-2-naphthylamine hydrochloride (23 g).
m.p. 257-262°C

¹H-NMR (300 MHz, DMSO-d₆): δ (ppm): 0.96 (t, 3H); 1.65-1.80 (m, 3H); 2.29 (m, 1H); 2.60 (m, 1H); 2.80-3.00 (m, 4H); 3.13 (dd, 1H); 3.34 (m, 1H); 3.68 (s, 3H); 3.77 (s, 3H); 6.83 (d, 1H); 6.89 (d, 1H). Mass: 250 [M+1].

### Example 3

### Preparation of (S)-N-propyl-5,6-dihydroxy-1,2,3,4-tetrahydro-2-naphthylamine hydrobromide

A solution of (S)-N-propyl-5,6-dimethoxy-1,2,3,4-tetrahydro-2-naphthylamine hydrochloride (22 g; 76.9 mmoles), prepared as described in example 2, in hydrobromic acid at 48% (220 ml) was heated under reflux (about 130°C) for 3 hours.

The solvent was evaporated to dryness under vacuum and the residue was collected twice with toluene, by evaporating to dryness.

The crude residue was collected with ethyl acetate and filtered obtaining (S)-N-propyl-5,6-dihydroxy-1,2,3,4-tetrahydro-2-naphthylamine hydrobromide (23 g).
m.p. 219-222°C
[α]_{D}=-54.59° (1% in methanol)

¹H-NMR (300 MHz, DMSO-d₆): δ (ppm): 0.93 (t, 3H); 1.68 (m, 3H); 2.25 (m, 1H); 2.40-2.55 (m, 1H); 2.70-3.10 (m, 5H); 3.31 (m, 1H); 6.40 (d, 1H); 6.61 (d, 1H).
Mass: 222 [M+1].

### Example 4

### Preparation of (R)-N-propyl-6,7-dihydroxy-1,2,3,4-tetrahydro-2-naphthylamine hydrochloride

Palladium on charcoal at 10% (50% in water; 0.5 g) and triethylamine (2.1 g; 21 mmoles) were added to a solution of (R)-6,7-dihydroxy-1,2,3,4-tetrahydro-2-naphthylamine hydrobromide (5 g; 19 mmoles) and propionaldehyde (1.1 g; 19 mmoles) in ethanol (150 ml).

The reaction mixture was kept under stirring and under hydrogen pressure (2.7 atm) at 35°C for 8 hours.

The catalyst was filtered off and the solvent evaporated under reduced pressure. The residue was dissolved in absolute ethanol (100 ml) and a solution of hydrochloric acid in ethyl ether (15% w/v) was added up to clearly acid pH.

The solvent was evaporated and the crude was purified by chromatography (eluent CH₂Cl₂:CH₃OH:CH₃COOH=90:10:1) obtaining (R)-N-propyl--6,7-dihydroxy-1,2,3,4-tetrahydro-2-naphthylamine hydrochloride (3.8 g) as a white solid.
m.p. 201-203°C

¹H-NMR (200 MHz, DMSO-d₆): δ (ppm): 0.93 (t, 3H); 1.56-1.78 (m, 3H); 2.19 (m, 1H); 2.53-3.02 (m, 6H); 3.30 (m, 1H); 6.46 (s, 2H). Mass: 222 [M+1].

### Example 5

### Preparation of 6-[(3-methoxyphenyl)acetylamino]hexanoic acid

N,N-dimethylformamide (0.1 ml) and thionyl chloride 13.5 g; 29.4 mmoles) were added under stirring at room temperature to a solution of 3-methoxyphenylacetic acid (3.3 g; 19.8 mmoles) in methylene chloride (50 ml).

After 1 hour, the solvent was evaporated under reduced pressure obtaining an oil which was dissolved in methylene chloride (6 ml). The resultant solution was added dropwise, contemporaneously to a solution of sodium hydroxide 4N (5 ml) and under vigorous stirring, to a solution of 6-aminohexanoic acid (2.5 g; 19.1 mmoles) and sodium hydroxide (0.8 g; 20 mmoles) in water (10 ml).

The reaction mixture was kept under stirring at room temperature for 3 hours.

The phases were separated and the aqueous phase was washed with methylene chloride (10 ml), acidified with hydrochloric acid at 37% up to pH 1 and extracted with methylene chloride (15 ml).

After drying the resultant organic phase on anhydrous sodium sulphate, the solvent was evaporated under reduced pressure.

The solid residue was triturated in ethyl ether obtaining after filtration 6-[(3-methoxyphenyl)acetylamino]hexanoic acid (4.4 g) as a white solid.
m.p. 75-76°C

¹H-NMR (200 MHz, CDCl₃): δ (ppm): 1.16-1.65 (m, 6H); 2.29 (t, 2H); 3.18 (m, 2H); 3.52 (s, 2H); 3.78 (s, 3H); 5.51 (bt, 1H); 6.74-7.29 (m, 4H).
Mass: 280 [M+1].

By working in a similar way the following compounds were prepared:

### 6-[(4-methoxypenyl)acetylamino]hexanoic acid

¹H-NMR (200 MHz, CDCl₃): δ (ppm): 1.16-1.66 (m, 6H); 2.28 (t, 2H); 3.17 (m, 2H); 3.49 (s, 2H); 3.79 (s, 3H); 5.43 (bt, 1H); 6.81-7.18 (m, 4H).
Mass: 280 [M+1].

### 6-[(3,4-dimethoxyphenyl)acetylamino]hexanoic acid

¹H-NMR (200 MHz, DMSO-d₆): δ (ppm): 1.12-1.51 (m, 6H); 2.17 (t, 2H); 2.99 (m, 2H); 3.28 (s, 2H); 3.69 (s, 3H); 3.70 (s, 3H); 6.70-6.89 (m, 3H); 7.94 (bt 1H).
Mass: 310 [M+1].

### 6-[(3.5-dimethoxyphenyl)acetylamino]hexanoic acid

¹H-NMR (200 MHz, CDCl₃): δ (ppm): 1.16-1.66 (m, 6H); 2.29 (t, 2H); 3.18 (m, 2H); 3.49 (s, 2H); 3.76 (s, 6H); 5.53 (bt, 1H); 6.37 (s, 3H).
Mass: 310 [M+1].

### 6-(phenylacetylamino)hexanoic acid

¹H-NMR (200 MHz, DMSO-d₆): δ (ppm): 1.11-1.54 (m, 6H); 2.16 (t, 3H); 3.00 (m, 2H); 3.36 (s, 2H); 7.11-7.32 (m, 5H); 8.00 (bt, 1H); 11.98 (bs, 1H).
Mass: 250 [M+1].

### 6-[(3,4-dimethoxyphenyl)acetylamino]octanoic acid

¹H-NMR (200 MHz, CDCl₃): δ (ppm): 1.11-1.67 (m, 10H); 2.30 (t, 2H); 3.16 (m, 2H); 3.49 (s, 2H); 3.85 (s, 6H); 5.43 (bt, 1H); 6.72-6.87 (m, 3H).

### 4-(phenylacetylamino)butanoic acid

¹H-NMR (200 MHz, CDCl₃): δ (ppm): 1.73 (m, 2H); 2.29 (t, 2H); 3.23 (m, 2H); 3.55 (s, 2H); 5.77 (bt, 1H); 7.19-7.39 (m, 5H).

### 6-(3-phenylpropionylamino)hexanoic acid

¹H-NMR (200 MHz, DMSO-d₆): δ (ppm): 1.18-1.25 (m, 2H); 1.31-1.38 (m, 2H); 1.42-1.49 (m, 2H); 2.13-2.20 (t, 2H); 2.31-2.38 (t, 2H); 2.76-2.83 (t, 2H); 2.99-3.02 (m, 2H); 7.17-7.25 (m, 5H); 7.79 (bs, 1H); 12.01 (bs, 1H).
Mass (electron impact): 263 [M⁺]

### Example 6

### Preparation of 6-[N-(2-phenylethyl)-N-methyl]amino-6-oxo-hexanoic acid

A solution of hexandioic acid monomethylester chloride (2.8 g; 15.7 mmoles) in methylene chloride (10 ml) was added to a solution of N-methyl-2-phenylethylamine (2 g; 14.8 mmoles) and 1,8-diazabicyclo-[5.4.0]undec-7-ene (3.6 g; 23.6 mmoles) in methylene chloride (20 ml), under stirring at room temperature.

After 2 hours water (30 ml) was added and the phases were separated. The organic phase was washed with an aqueous solution of hydrochloric acid 0.2 N, then with a saturated aqueous solution of sodium chloride and dried on anhydrous sodium sulphate.

After evaporation of the solvent under reduced pressure, the residue was dissolved in methanol (12 ml).

A solution of sodium hydroxide (1.6 g; 40 mmoles) in water (8 ml) was added dropwise to the solution, under stirring at room temperature.

The reaction mixture was kept under stirring for 3 hours.

After evaporation of the solvent under reduced pressure, the residue was dissolved in water (10 ml).

The solution was washed with ethyl ether (10 ml), then acidified with hydrochloric acid at 37% up to pH 1 and extracted with methylene chloride (20 ml).

The organic phase was dried on anhydrous sodium sulphate.

After evaporation of the solvent, 6-[N-(2-phenylethyl)-N-methyl]-amino-6-oxo-hexanoic acid (3.6 g) was obtained as an oil.

¹H-NMR (200 MHz, DMSO-d₆): δ (ppm): 1.26-1.58 (m, 4H); 1.97-2.30 (m, 4H): 2.66-2.83 (m, 2H); 2.79 and 2.88 (2s, 3H); 3.40-3.55 (m, 2H); 7.12-7.35 (m, 5H); 11.98 (bs, 1H).
Mass: 264 [M+1].

By working in a similar way the following compound was prepared:

### 6-[2-(4-methoxyphenyl)ethylamino]-6-oxo-hexanoic acid

¹H-NMR (200 MHz, DMSO-d₆): δ (ppm): 1.35-1.53 (m, 4H); 1.96-2.08 (m, 2H); 2.12-2.24 (m, 2H); 2.61 (t, 2H); 3.12-3.27 (m, 2H); 3.70 (s, 3H); 6.80-7.13 (m, 4H); 7.84 (t, 1H); 12.01 (s, 1H).
Mass: 280 [M+1].

### Example 7

### Preparation of (S)-N-propyl-N-[6-[2-(3-methoxyphenyl)ethylamino]hexyl]-5,6-dimethoxy-1,2,3,4-tetrahydro-2-naphthylamine dihydrochloride

Thionyl chloride (0.82 g; 6.9 mmoles) was added to a solution of 6-[(3-methoxyphenyl)acetylamino]hexanoic acid (1.4 g; 5.3 mmoles), prepared as described in example 5, in methylene chloride (10 ml), under stirring at room temperature.

After 1 hour the solvent was evaporated under reduced pressure obtaining an oil which was dissolved in methylene chloride (6 ml). The resultant solution was added dropwise to a solution prepared by adding, under stirring at room temperature, triethylamine (1.6 g; 15.8 mmoles) to a suspension of (S)-N-propyl-5,6-dimethoxy-1,2,3,4-tetrahydro-2-naphthylamine hydrobromide (1.7 g; 5.1 mmoles), prepared in a way similar to that described in example 2, in methylene chloride (20 ml).

The reaction mixture was kept under stirring at room temperature for 1 hour and then water (40 ml) was added.

The phases were separated and the organic phase was washed with water, dried on anhydrous sodium sulphate and brought to dryness under reduced pressure.

The resultant residue was dissolved under nitrogen in tetrahydrofuran (20 ml) and borane-dimethylsulphide complex (1.9 g; 24.4 mmoles) was slowly added to the solution, under stirring at room temperature.

At the end of the addition, the mixture was heated under reflux for 1.5 hours.

After cooling at 5°C a solution of hydrochloric acid 37% (1.2 ml) in methanol (15 ml) was added.

The mixture was heated again under reflux for 1 hour, then concentrated by distilling the solvents at atmospheric pressure and brought to dryness under reduced pressure.

The resultant residue was dissolved in methanol (20 ml). The solvent was distilled under reduced pressure and methanol (20 ml) was added again to the residue.

After evaporation of the solvent to dryness, the resultant oily residue was purified by chromatography (eluent CH₂Cl₂:CH₃OH:HCOOH 50%=90:10:1).

After dissolution in absolute ethanol (20 ml) and addition of a solution of hydrochloric acid in ethyl ether (15% w/v) up to clearly acid pH, the solvents were evaporated under reduced pressure obtaining (S)-N-propyl-N-[6-[2-(3-methoxyphenyl)ethylamino]hexyl]-5,6-dimethoxy-1,2,3,4-tetrahydro-2-naphthylamine dihydrochloride (2.2 g) as an amorphous white solid.

¹H-NMR (200 MHz, CDCl₃): δ (ppm): 0.96 (t, 3H); 1.35-2.57 (m, 12H); 2.53-3.36 (m, 14H); 3.42-3.63 (m, 1H); 3.72 (s, 3H); 3.73 (s, 3H); 3.78 (s, 3H); 6.66-7.19 (m, 6H); 9.67 (bs, 2H); 10.88 (bs, 1H). Mass: 483 [M+1].

By working in a similar way the following compounds were prepared:

### (S)-N-propyl-N-[6-[2-(4-methoxylphenyl)ethylamino]hexyl]-5,6-dimethoxy-1,2,3,4-tetrahydro-2-naphthylamine dihydrochloride

¹H-NMR (200 MHz, D₂O): δ (ppm): 0.81 (t, 3H); 1.13-2.20 (m, 12H); 2.43-3.19 (m, 14H); 3.40-3.60 (m, 1H); 3.58 (s, 3H); 3.62 (s, 3H); 3.67 (s, 3H); 6.72-7.13 (m, 6H).
Mass: 483 [M+1].

### (S)-N-propyl-N-[6-[2-(3,4-dimethoxyphenyl)ethylamino]hexyl]-5,6-dimethoxy-1,2,3,4-tetrahydro-2-naphthylamine dihydrochloride

¹H-NMR (200 MHz, CDCl₃): δ (ppm): 0.98 (t, 3H); 1.32-2.60 (m, 12H); 2.52-3.40 (m, 14H); 3.45-3.70 (m, 1H); 3.72 (s, 3H); 3.77 (s, 3H); 3.78 (s, 3H); 3.80 (s, 3H); 6.66-6.84 (m, 5H); 9.71 (bs, 2H); 10.93 (bs, 1H).

### (S)-N-propyl-N-[6-[2-(3,5-dimethoxyphenyl)ethylamino]hexyl]-5,6-dimethoxy-1,2,3,4-tetrahydro-2-naphthylamine dihydrochloride

¹H-NMR (200 MHz, CDCl₃): δ (ppm): 0.87 (t, 3H); 1.20-2.14 (m, 12H); 2.46-3.14 (m, 15H); 3.74 (s, 6H); 3.77 (s, 3H); 3.81 (s, 3H); 6.27-6.81 (m, 5H).

### (S)-N-methyl-N-(2-phenylmethyl)-N'-propyl-N'-[2-(5,6-dimethoxy-1,2,3,4-tetrahydro)naphthyl]-1,6-hexandiamine dihydrochloride

¹H-NMR (200 MHz, CDCl₃): δ (ppm): 0.98 (t, 3H); 1.37-2.53 (m, 12H); 2.56-3.40 (m, 14H); 2.83 (s, 3H); 3.50-3.70 (m, 1H); 3.75 (s, 3H); 3.79 (s, 3H); 6.72 (d, 1H); 6.81 (d, 1H); 7.16-7.32 (m, 5H).
Mass: 467 [M+1].

### (S)-N-propyl-N-[8-[2-(3,4-dimethoxyphenyl)ethylamino]octyl]-5,6-dimethoxy-1,2,3,4-tetrahydro-2-naphthylamine dihydrochloride

¹H-NMR (200 MHz, CDCl₃): δ (ppm): 1.00 (t, 3H); 1.17-2.56 (m, 16H); 2.54-3.33 (m, 14H); 3.45-3.70 (m, 1H); 3.72 (s, 3H); 3.78 (s, 3H); 3.79 (s, 3H); 3.80 (s, 3H); 6.66-6.86 (m, 5H); 8.44 (bs, 1H); 9.68 (bs, 2H).

### Example 8

### Preparation of (S)-N-propyl-N-[6-[2-(3,4-dimethoxyphenyl)ethylamino]hexyl]-5-methoxy-1,2,3,4-tetrahydro-2-naphthylamine dihydrochloride

By working in a way similar to that described in example 7 but using as starting compound (S)-N-propyl-5-methoxy-1,2,3,4-tetrahydro-2--naphthylamine [J. Med. Chem., 29, 912 (1986)], (S)-N-propyl-N-[6--[2-(3,4-dimethoxyphenyl)ethylamino]hexyl]-5-methoxy-1,2,3,4-tetrahydro-2-naphthylamine dihydrochloride was prepared.

¹H-NMR (200 MHz, CDCl₃): δ (ppm): 0.86 (t, 3H); 1.22-2.11 (m, 12H); 2.40-3.14 (m, 14H); 3.79-3.84 and 3.86 (3s, 9H); 6.60-7.11 (m, 6H).
Mass: 483 [M+1].
Analogously, the following compound was prepared:

### (S)-N-propyl-N-[6-[2-(4-methoxyphenyl)ethylamino]hexyl]-5-methoxy-1,2,3,4-tetrahydro-2-naphthylamine dihydrochloride

¹H-NMR (200 MHz, D₂O): δ (ppm): 0.82 (t, 3H); 1.12-2.21 (m, 12H); 2.31-3.18 (m, 14H); 3.40-3.61 (m, 1H); 3.65 (s, 3H); 3.67 (s, 3H); 6.63-7.12 (m, 7H).
Mass: 453 [M+1].

### Example 9

### Preparation of (S)-N-propyl-N-[4-(2-phenylethylamino)butyl]-5,6-dimethoxy-1,2,3,4-tetrahydro-2-naphthylamine dihydrochloride

A solution of 4-(phenylacetylamino)butanoic acid (2.6 g; 10.8 mmoles) , prepared as described in example 5, and of N,N'-dicyclohexylcarbodiimide (2.2 g; 10.8 mmoles) in chloroform (100 ml) was kept under stirring at 5°C for 30 minutes.

Triethylamine (1.1 g; 10.8 mmoles) and, portionwise, (S)-N-propyl--5,6-dimethoxy-1,2,3,4-tetrahydro-2-naphthylamine hydrobromide (3 g; 9.1 mmoles), prepared in a way similar to that described in example 2, were then added.

At the end of the addition the temperature was allowed to arise up to the room value.

After 2 hours the reaction mixture was filtered on a porous septum and the resultant solution was brought to dryness under reduced pressure.

The residue was collected with acetone and, after filtration, the solution was brought again to dryness under reduced pressure. The residue was dissolved in methylene chloride and the resultant solution was washed with a 10% aqueous solution of sodium bicarbonate, with a 5% aqueous solution of hydrochloric acid and then with a saturated aqueous solution of sodium chloride.

After drying on anhydrous sodium sulphate and evaporation of the solvent under reduced pressure, the resultant oil was dissolved under nitrogen in tetrahydrofuran (30 ml).

Borane-dimethylsulphide complex (4.7 g; 60.1 mmoles) was slowly added to the solution, under stirring at room temperature.

At the end of the addition the reaction mixture was heated under reflux for 1.5 hours.

After cooling at 5°C a 37% solution of hydrochloric acid (1.6 ml) in methanol (15 ml) was added.

The reaction mixture was again heated under reflux for 1 hour, then concentrated by distilling the solvents at atmospheric pressure and brought to dryness under reduced pressure.

The residue was dissolved in methanol (30 ml), the solvent was distilled under reduced pressure, methanol (30 ml) was added again and the solvent was distilled again to dryness.

After adding absolute ethanol (30 ml) and a solution of hydrochloric acid in ethyl ether (15% w/v) (1 ml), the solvents were evaporated obtaining (S)-N-propyl-N-[4-12-phenylethylamino)butyl]-5,6-dimethoxy-1,2,3,4-tetrahydro-2-naphthylamine dihydrochloride (4 g) as an amorphous white solid.

¹H-NMR (200 MHz, DMSO-d₆): δ (ppm): 0.91 (t, 3H); 1.07-2.46 (m, 8H); 2.51-3.31 (m, 14H); 3.44-3.65 (m, 1H); 3.66 (s, 3H); 3.74 (s, 3H); 6.82 (d, 1H); 6.89 (d, 1H); 7.18-7.37 (m, 5H).

### Example 10

### Preparation of (S)-N-propyl-N-[6-[2-(3-hydroxyphenyl)ethylamino)-hexyl]-5,6-dihydroxy-1,2,3,4-tetrahydro-2-naphthylamine dihydrobromide (Compound 1)

A solution of (S)-N-propyl-N-[6-[2-(3-ethoxyphenyl)ethylamino)-hexyl]-5,6-dimethoxy-1,2,3,4-tetrahydro-2-naphthylamine dihydrochloride (2.2 g; 3.9 mmoles), prepared as described in example 7, in 48% hydrobromic acid (25 ml) was heated under reflux under nitrogen for 4.5 hours.

The reaction mixture was then brought to dryness under reduced pressure and toluene (40 ml) was added to the resultant residue.

After evaporation of the solvent, absolute ethanol (40 ml) was added and the solvent was evaporated again.

The residue was purified by chromatography (eluent CH₂Cl₂:CH₃OH:HCOOH 50%=85:15:2) obtaining Compound 1 (2.0 g) as an amorphous white solid.

¹H-NMR (200 MHz, D₂O): δ (ppm): 0.80 (t, 3H); 1.17-2.17 (m, 12H); 2.36-3.15 (m, 14H); 3.42-3.56 (m, 1H); 6.46-7.13 (m, 6H).
Mass: 441 [M+1].

By working in a similar way the following compounds were prepared:

### (S)-N-propyl-N-[6-[2-(4-hydroxyphenyl)ethylamino)hexyl]-5,6-dihydroxy-1,2,3,4-tetrahydro-2-naphthylamine dihydrobromide (Compound 2)

¹H-NMR (200 MHz, D₂O): δ (ppm): 0.80 (t, 3H); 1.16-2.19 (m, 12H); 2.38-3.12 (m, 14H); 3.42-3.59 (m, 1H); 6.49 (d, 1H); 6.61 (d, 1H); 6.66-7.05 (m, 4H).
Mass: 441 [M+1].

### (S)-N-propyl-N-[6-[2-(3,4-dihydroxyphenyl)ethylamino)hexyl]-5,6-dihydroxy-1,2,3,4-tetrahydro-2-naphthylamine dihydrobromide (Compound 3)

¹H-NMR (200 MHz, D₂O): δ (ppm): 0.82 (t, 3H); 1.18-2.17 (m, 12H); 2.38-3.12 (m, 14H); 3.41-3.56 (m, 1H); 6.47-6.73 (m, 5H).
Mass: 457 [M+1].

### (S)-N-propyl-N-[6-[2-(3,5-dihydroxyphenyl)ethylamino)hexyl]-5,6-dihydroxy-1,2,3,4-tetrahydro-2-naphthylamine dihydrobromide (Compound 4)

¹H-NMR (200 MHz, D₂O): δ (ppm): 0.81 (t, 3H); 1.16-2.18 (m, 12H); 2.39-3.14 (m, 14H); 3.43-3.59 (m, 1H); 6.14-6.21 (m, 3H); 6.49 (d, 1H); 6.62 (d, 1H).
Mass: 457 [M+1].

### (S)-N-methyl-N-(2-phenylethyl)-N'-propyl-N'-[2-(5,6-dihydroxy-1,2,3,4-tetrahydro)naphthyl]-1.6-hexandiamine dihydrobromide (Compound 5)

¹H-NMR (200 MHz, D₂O): δ (ppm): 0.80 (t, 3H); 1.15-2.17 (m, 12H); 2.36-3.38 (m, 14H); 2.72 (3, 3H); 3.39-3.55 (m, 1H); 6.48 (d, 1H); 6.61 (d, 1H); 7.11-7.27 (m, 5H).
Mass: 439 [M+1].

### (S)-N-propyl-N-[6-[2-(3,4-dihydroxyphenyl)ethylamino]hexyl]-5-hydroxy-1,2,3,4-tetrahydro-2-naphthylamine dihydrobromide (Compound 6)

¹H-NMR (200 MHz, D₂O): δ (ppm): 0.82 (t, 3H); 1.19-2.22 (m, 12H); 2.38-3.21 (m, 14H); 3.49-3.65 (m, 1H); 6.55-7.00 (m, 6H).
Mass: 441 [M+1].

### (S)-N-propyl-N-[8-[2-(3,4-dihydroxyphenyl)ethylamino]octyl]-5-hydroxy-1,2,3,4-tetrahydro-2-naphthylamine dihydrobromide (Compound 7)

¹H-NMR (200 MHz, D₂O): δ (ppm): 0.79 (t, 3H); 1.13-2.18 (m, 16H); 2.68 (m, 2H); 2.37-3.09 (m, 10H); 3.01-3.09 (m, 2H); 3.40-3.56 (m, 1H); 6.46-6.71 (m, 5H).
Mass: 485 [M+1].

### (S)-N-propyl-N-[4-(2-phenyletylamino)butyl]-5,6-dihydroxy-1,2,3,4--tetrahydro-2-naphthylamine dihydrobromide (Compound 8)

¹H-NMR (200 MHz, D₂O): δ (ppm): 0.80 (t, 3H); 1.48-2.15 (m, 8H); 2.36-3.20 (m, 14H); 3.41-3.56 (m, 1H); 6.48 (d, 1H); 6.61 (d, 1H); 7.13-7.28 (m, 5H).
Mass: 397 [M+1].

### (S)-N-propyl-N-[6-[2-(4-hydroxyphenyl)ethylamino]hexyl]-5-hydroxy-1,2,3,4-tetrahydro-2-naphthylamine dihydrobromide (Compound 9)

¹H-NMR (200 MHz, D₂O): δ (ppm): 0.82 (t, 3H); 1.17-2.22 (m, 12H); 2.36-3.13 (m, 14H); 3.49-3.65 (m, 1H); 6.59-6.99 (m, 3H); 6.68-7.07 (m, 4H).
Mass: 425 [M+1].

### Example 11

### Preparation of (S)-N-propyl-N-[6-(2-phenylethylamino)hexyl]-5,6-dihydroxy-1,2,3,4-tetrahydro-2-naphthylamine dihydrochloride (Compound 10)

Thionyl chloride (1.4 g; 11.7 mmoles) was added to a solution of 6-(phenylacetylamino)hexanoic acid (1.1 g; 4.4 mmoles), prepared as described in example 5, in methylene chloride (9 ml), under stirring at room temperature.

After 1.5 hours the solvent was evaporated under reduced pressure obtaining a yellow oil which was dissolved in methylene chloride (8 ml).

Sodium tetraborate (1.3 g; 6.6 mmoles) was added to a solution of (S)-N-propyl-5,6-dihydroxy-1,2,3,4-tetrahydro-2-naphthylamine hydrobromide (1 g; 3.3 mmoles), prepared as described in example 3, in water (20 ml), under nitrogen at room temperature.

The mixture was heated under stirring at 70°C up to complete dissolution and then cooled at room temperature.

Methylene chloride (2 ml), potassium carbonate (3.6 g; 26 mmoles) and, under vigorous stirring, the previously prepared solution in methylene chloride were then added.

After 45 minutes at room temperature, 37% hydrochloric acid was added up to pH 1.

The phases were separated and the aqueous phase was extracted twice with methylene chloride (2x15 ml).

The collected organic phases, washed with brine slightly acidified with hydrochloric acid, were dried on anhydrous sodium sulphate.

After filtration and evaporation to dryness, the solid residue was dissolved under nitrogen in tetrahydrofuran (14 ml).

Borane-dimethylsulphide complex (3.4 g; 42.4 mmoles) was slowly added to the resultant solution under stirring.

At the end of the addition the mixture was heated under reflux for 1.5 hours.

After cooling at 5-10°C a 37% hydrochloric acid solution (1.5 ml) in methanol (13 ml) was added.

The reaction mixture was heated again under reflux for 1 hour, then concentrated by distilling the solvents at atmospheric pressure and brought to dryness under reduced pressure.

The resultant residue was dissolved in methanol (17 ml) and the solvent was distilled under reduced pressure.

After adding absolute ethanol (17 ml), the solvent was evaporated again up to dryness.

The residue was dissolved in absolute ethanol (17 ml) and a solution of hydrochloric acid in ethyl ether (15% w/v; 1 mi) was added.

After evaporation of the solvents, the residue was purified by chromatography (eluent CH₂Cl₂:CH₃OH:HCOOH 50%=80:15:1).

The resultant solid was dissolved in absolute ethanol (20 ml) and, after addition of a solution of hydrochloric acid in ethyl ether (15% w/v) up to clearly acid pH, the solvents were evaporated under reduced pressure.

Compound 10 (0.7 g) was obtained as a white amorphous solid.

¹H-NMR (200 MHz, D₂O): δ (ppm): 0.78 (t, 3H); 1.15-1.64 (m, 10H); 1.40-2.15 (m, 2H); 2.37-3.07 (m, 12H); 3.08-3.16 (m, 2H); 3.42-3.56 (m, 1H); 6.47 (d, 1H); 6.60 (d, 1H); 7.10-7.27 (m, 5H).
Mass: 425 [M+1].

By working in a similar way the following compounds were prepared:

### (S)-N-propyl-N-[6-(2-phenylethylamino)hexyl]-6,7-dihydroxy-1,2,3,4--tetrahydro-2-naphthylamine dihydrochloride (Compound 11)

¹H-NMR (200 MHz, D₂O): δ (ppm): 0.80 (t, 3H); 1.16-2.10 (m, 12H); 2.49-3.09 (m, 12H); 3.10-3.18 (m, 2H); 3.44-3.61 (m, 1H); 6.51 (s, 2H); 7.13-7.28 (m, 5H).
Mass: 425 [M+1].

### (S)-N-propyl-N-[6-(3-phenylpropylamino)hexyl]-5,6-dihydroxy-1,2,3,4--tetrahydro-2-naphthylamine dihydrochloride (Compound 12)

¹H-NMR (200 MHz, D₂O): δ (ppm): 0.81 (t, 3H); 1.23-2.18 (m, 14H); 2.39-3.04 (m, 14H); 3.44-3.58 (m, 1H); 6.50 (d, 1H); 6.63 (d, 1H); 7.08-7.25 (m, 5H).
Mass: 439 [M+1].

### (R)-N-propyl-N-[6-[2-(3,4-dimethoxyphenyl)ethylamino]hexyl]-6,7-dihydroxy-1,2,3,4-tetrahydro-2-naphthylamine dihydrochloride

¹H-NMR (200 MHz, DMSO-d₆): δ (ppm): 0.91 (t, 3H); 1.18-2.32 (m, 12H); 2.58-3.60 (m, 15H); 3.70 (s, 3H); 3.74 (s, 3H); 6.43-6.89 (m, 5H); 8.90-9.10 (bs, 2H); 10.09-10.20 (bs, 1H).

### (R)-N-propyl-N-[6-[2-(4-methoxyphenyl)ethylamino]hexyl]-6,7-dihydroxy-1,2,3,4-tetrahydro-2-naphthylamine dihydrochloride

¹H-NMR (200 MHz, DMSO-d₆): δ (ppm): 0.92 (t, 3H); 1.21-2.31 (m, 12H); 2.57-3.61 (m, 15H); 3.72 (s, 3H); 6.46 (s, 1H); 6.48 (s, 1H); 6.84-7.21 (m, 4H); 8.60-8.91 (bs, 2H); 8.88-9.11 (bs, 2H); 10.09-1 0.23 (bs, 1H).
Mass: 455 [M+1]

### Example 12

### Preparation of (R)-N-propyl-N-[6-[2-(3,4-dihydroxyphenyl)ethylamino]hexyl]-6,7-dihydroxy-1,2,3,4-tetrahydro-2-naphthylamine dihydrobromide (Compound 13)

A solution of (R)-N-propyl-N-[6-[2-(3,4-dimethoxyphenyl)ethylamino]-hexyl]-6,7-dihydroxy-1,2,3,4-tetrahydro-2-naphthylamine dihydrochloride (2.5 g; 4.5 mmoles), prepared as described in example 11, in 48% hydrobromic acid (20 ml) was heated under reflux for 5 hours. The reaction mixture was brought to dryness under reduced pressure and absolute ethanol (30 ml) was added to the residue.

After evaporation to dryness, absolute ethanol (30 ml) was added again and the solvent was evaporated under reduced pressure.

The residue was purified by chromatography (eluent CH₂Cl₂:CH₃OH:HCOOH 50%=80:20:4) obtaining Compound 13 (1.8 g) as a white amorphous solid.

¹H-NMR (200 MHz, D₂O): δ (ppm): 0.79 (t, 3H); 1.13-2.06 (m, 12H); 2.46-3.10 (m, 14H); 3.40-3.56 (m, 1H); 6.48-6.71 (m, 5H).
Mass: 457 [M+1].

By working in a similar way the following compound was prepared:

### (R)-N-propyl-N-[6-[2-(4-hydroxyphenyl)ethylamino]hexyl]-6,7-dihydroxy-1,2,3,4-tetrahydro-2-naphthylamine dihydrobromide (Compound 14)

¹H-NMR (200 MHz, D₂O): δ (ppm): 0.79 (t, 3H); 1.16-2.07 (m, 12H); 2.48-3.11 (m, 14H); 3.45-3.59 (m, 1H); 6.51 (s, 2H); 6.66-7.04 (m, 4H).
Mass: 441 [M+1]

### Example 13

### Preparation of (S)-N-propyl-N-[(6-phthalimido-1-oxo)hexyl]-5,6-dimethoxy-1,2,3,4-tetrahydro-2-naphthylamine

Triethylamine (12.7 g; 126.1 mmoles) and then a solution of 6--phthalimidohexanoic acid chloride (15.5 g; 55.5 mmoles) in methylene chloride (120 ml) were added to a suspension of (S)-N-propyl-5,6-dimethoxy-1,2,3,4-tetrahydro-2-naphthylamine hydrochloride (14.4 g; 50.4 mmoles), prepared as described in example 2, in methylene chloride (150 ml) kept under stirring at room temperature.

The reaction mixture was kept under stirring at room temperature for 1.5 hours.

After addition of water (250 ml) and separation of the phases, the organic phase was washed with water (150 ml), dried on anhydrous sodium sulphate and the solvent was evaporated under reduced pressure.

The residue was purified by chromatography (eluent petroleum ether: ethyl acetate=6:4) obtaining (S)-N-propyl-N-[(6-phthalimido-1-oxo)-hexyl]-5,6-dimethoxy-1,2,3,4-tetrahydro-2-naphthylamine (24.1 g).

¹H-NMR (200 MHz, CDCl₃): δ (ppm): 0.80-0.94 (2t, 3H); 1.30-2.02 (m, 10H); 2.26-2.38 (m, 2H); 2.59-3.22 (m, 6H); 3.60-3.72 (m, 2H); 3.75-3.84 (45, 6H); 3.85-4.66 (m, 1H); 6.66-6.82 (m, 2H); 7.64-7.85 (m, 4H).
Mass: 493 [M+1].

### Example 14

### Preparation of (S)-N-propyl-N-[(6-amino-1-oxo)hexyl]-5,6-dimethoxy--1,2,3,4-tetrahydro-2-naphthylamine

A solution of (S)-N-propyl-N-[(6-phthalimido-1-oxo)hexyl]-5,6-dimethoxy-1,2,3,4-tetrahydro-2-naphthylamine (24.1 g; 48.9 mmoles), prepared as described in example 13, in 33% ethanolic methylamine (240 ml) was kept under stirring at room temperature for 20 hours. The reaction mixture was brought to dryness under reduced pressure and the residue was purified by chromatography (eluent methylene chloride:methanol:ammonia 30%=90:10:1) obtaining (S)-N-propyl-N-[(6-amino-1-oxo)hexyl]-5,6-dimethoxy-1,2,3,4-tetrahydro-2-naphthylamine (11.9 g) as an oil.

¹H-NMR (200 MHz, CDCl₃): δ (ppm): 0.80-0.93 (2t, 3H); 1.20-2.04 (m, 10H); 2.25-2.48 (m, 2H); 2.58-3.21 (m, 8H); 3.72-3.81 (45, 6H); 3.82-4.64 (m, 1H); 6.66-6.80 (m, 2H).
Mass: 363 [M+1].

### Example 15

### Preparation of (S)-N-propyl-N-[6-[2-(2-methoxyphenyl)ethylamino]-hexyl]-5,6-dimethoxy-1,2,3,4-tetrahydro-2-naphthylamine dihydrochloride

Triethylamine (0.83 g; 8.3 mmoles) and then a solution of (2-methoxyphenyl) acetyl chloride (1.1 g; 6 mmoles) in methylene chloride (10 ml) were added to a solution of (S)-N-propyl-N-[(6-amino-1-oxo)-hexyl]-5,6-dimethoxy-1,2,3,4-tetrahydro-2-naphthylamine (2 g; 5.5 mmoles), prepared as described in example 14, in methylene chloride (20 ml), kept under stirring at room temperature.

The reaction mixture was kept under stirring at room temperature for 3 hours.

After addition of water (30 ml) and separation of the phases, the organic phase was washed with water, dried on anhydrous sodium sulphate and the solvent was evaporated under reduced pressure.

The residue was dissolved under nitrogen in tetrahydrofuran (10 ml) and borane-dimethylsulphide complex (1.9 g; 23.3 mmoles) was slowly added at room temperature to the resultant solution, under stirring and under nitrogen.

At the end of the addition the reaction mixture was heated under reflux for 1.5 hours.

After cooling at 5-10°C a 37% hydrochloric acid solution (1 mi) in methanol (7 ml) was added.

The reaction mixture was heated again under reflux for 1 hour, then concentrated by distilling the solvents at atmospheric pressure and brought to dryness under reduced pressure.

The resultant residue was dissolved in methanol (30 ml) and the solvent was distilled under reduced pressure. Methanol (30 ml) was added again and the solvent was distilled to dryness again.

The residue was dissolved in absolute ethanol (30 ml) and a solution of hydrochloric acid in ethyl ether (15% w/v) (10 ml) was added.

After evaporation of the solvents, (S)-N-propyl-N-[6-[2-(2-methoxyphenyl)ethylamino]hexyl]-5,6-dimethoxy-1,2,3,4-tetrahydro-2-naphthylamine dihydrochloride (1.7 g) was obtained as a white amorphous solid.

¹H-NMR (200 MHz, D₂O): δ (ppm): 0.80 (t, 3H); 1.15-2.20 (m, 12H); 2.45-3.65 (m, 15H); 3.58 (s, 3H); 3.68 (s, 6H); 6.78-7.22 (m, 6H).
Mass: 483 [M+1].

### Example 16

### Preparation of (S)-N-propyl-N-[6-[2-(2-hydroxyphenyl)ethylamino]hexyl]-5,6-dihydroxy-1,2,3,4-tetrahydro-2-naphthylamine dihydrobromide (Compound 15)

A solution of (S)-N-propyl-N-[6-[2-(2-methoxyphenyl)ethylamino]-hexyl]-5,6-dimethoxy-1,2,3,4-tetrahydro-2-naphthylamine dihydrochloride (1.7 g; 3 mmoles), prepared as described in example 15, in 48% hydrobromic acid (17 ml) was heated under reflux under nitrogen for 4 hours.

The reaction mixture was then brought to dryness under reduced pressure and absolute ethanol (30 ml) was added to the resultant residue.

After evaporation of the solvent, ethyl acetate (30 ml) was added and the solvent was evaporated again under reduced pressure.

The crude was purified by chromatography (eluent CH₂Cl₂:CH₃OH:HCOOH 50%=85:15:1) obtaining Compound 15 (0.83 g) as a white amorphous solid.

¹H-NMR (200 MHz, D₂O): δ (ppm): 0.81 (t, 3H); 1.13-2.21 (m, 12H); 2.38-3.15 (m, 14H); 3.44-3.59 (m, 1H); 6.49 (d, 1H); 6.62 (d, 1H); 6.73-7.01 (m, 4H).
Mass: 441 [M+1].

### Example 17

### Preparation of (S)-N-propyl-N-[6-(2-amino)hexyl]-5,6-dimethoxy--1,2,3,4-tetrahydro-2-naphthylamine dihydrochloride

Borane-dimethylsulphide complex (3 g; 37 mmoles) was slowly added at room temperature, under stirring and under nitrogen, to a solution of (S)-N-propyl-N-[(6-amino-1-oxo)hexyl]-5,6-dimethoxy-1,2,3,4--tetrahydro-2-naphthylamine (2.3 g; 6.34 mmoles), prepared as described in example 14, in tetrahydrofuran (40 ml).

At the end of the addition the reaction mixture was heated under reflux for 2 hours.

After cooling at 5°C a 37% hydrochloric acid solution (1.5 ml) in methanol (12 ml) was added.

The reaction mixture was heated again under reflux for 1 hour, then concentrated by distilling the solvents at atmospheric pressure and by bringing to dryness under reduced pressure.

The residue was dissolved in methanol (30 ml) and the solvent distilled under reduced pressure; methanol (30 ml) was added again and the solvent was evaporated to dryness.

The crude was purified by chromatography (eluent CH₂Cl₂:CH₃OH:HCOOH 50%=85:15:1).

The resultant solid was dissolved in absolute ethanol and a solution of hydrochloric acid in ethyl ether (15% w/v) was added up to clearly acid pH.

By evaporation of the solvents under reduced pressure (S)-N-propyl-N-[6-(2-amino)hexyl]-5,6-dimethoxy-1,2,3,4-tetrahydro2-naphthylamine dihydrochloride (1.9 g) was obtained as a white amorphous solid.

¹H-NMR (200 MHz, D₂O): δ (ppm): 0.80 (t, 3H); 1.19-1.32 (m, 4H); 1.40-2.20 (m, 8H); 2.44-3.17 (m, 10H); 3.46-3.63 (m, 1H); 3.59 (s, 3H); 3.68 (s, 3H); 6.76-6.85 (2d, 2H).
Mass: 349 [M+1].

### Example 18

### Preparation of (S)-N,N'-dipropyl-N-[2-(5,6-dimethoxy-1,2,3,4-tetrahydro)naphthyl]-1,6-hexandiamine

Palladium on charcoal 10% (50% water; 0.3 g) was added to a solution of (S)-N-propyl-N-[6-12-amino)hexyl]-5,6-dimethoxy-1,2,3,4-tetrahydro-2-naphthylamine dihydrochloride (1.5 g; 4.3 mmoles), prepared as described in example 17, and of propionaldehyde (0.25 g; 4.4 mmoles) in ethanol (30 ml).

The reaction mixture was kept under stirring and under hydrogen pressure (2.7 atm) at room temperature for 8 hours.

The catalyst was filtered and the solvent evaporated under reduced pressure.

The crude was purified by chromatography (eluent methylene chloride: methanol:ammonia 30%=90:10:0.5) obtaining (S)-N,N'-dipropyl-N-[2-(5,6-dimethoxy-1,2,3,4-tetrahydro)naphthyl]-1,6-hexandiamine (1.3 g) as an oil.

¹H-NMR (200 MHz, CDCl₃): δ (ppm): 0.85 (t, 3H); 0.89 (t, 3H); 1.22 -2.07 (m, 14H); 2.38-3.13 (m, 13H); 3.77 (s, 3H); 3.81 (s, 3H); 6.70 (d, 1H); 6.78 (d, 1H).
Mass: 391 [M+1].

### Example 19

### Preparation of (S)-N-propyl-N-[2-(4-methoxyphenyl)ethyl]-N'-propyl-N'-[2-(5,6-dimethoxy-1,2,3,4-tetrahydro)naphthyl]-1,6-hexandiamine

1,8-Diazabicyclo[5.4.0]undec-7-ene (2.2 g; 14.4 mmoles) and then a solution of (4-methoxyphenyl)acetyl chloride (0.7 g; 3.8 mmoles) in methylene chloride (5 ml) were added to a solution of (S)-N,N'-dipropyl-N-[2-(5,6-dimethoxy-1,2,3,4-tetrahydro)naphthyl]-1,6-hexandiamine (1.3 g; 3.3 moles), prepared as described in example 18, in methylene chloride (13 ml), kept under stirring at room temperature. The reaction mixture was kept under stirring at room temperature for 3 hours.

After addition of water (30 ml) and separation of the phases, the organic phase was washed with water, dried on anhydrous sodium sulphate and the solvent evaporated under reduced pressure.

The residue was dissolved under nitrogen in tetrahydrofuran (19 mi) and borane-dimethylsulphide complex (1,2 g; 15 mmoles) was slowly added at room temperature to the resultant solution.

At the end of the addition the reaction mixture was heated under reflux for 1.5 hours.

After cooling at 5-10°C a 37% hydrochloric acid solution (1 mi) in methanol (5 ml) was added.

The reaction mixture was heated again under reflux for 1 hour, then concentrated by distilling the solvents at atmospheric pressure and brought to dryness under reduced pressure.

The resultant residue was dissolved in methanol (30 ml) and the solvent was distilled under reduced pressure; methanol (30 ml) was added again and the solvent was evaporated again up to dryness.

Then the residue was dissolved in absolute ethanol (30 mi) and the solvent evaporated to dryness.

The crude was purified by chromatography (eluent methylene chloride: methanol:ammonia 30%=97:3:0.1) obtaining (S)-N-propyl-N-[2-(4-methoxyphenyl)ethyl]-N'-propyl-N'-[2-(5,6-dimethoxy-1,2,3,4-tetrahydro)-naphthyl]-1,6-hexandiamine (1 g) as an oil.

¹H-NMR (200 MHz, CDCl₃); δ (ppm): 0.91 (t, 6H); 1.20-1.80 (m, 13H); 2.18-3.30 (m, 18H); 3.76 (s, 6H); 3.81 (s, 3H); 6.70-7.17 (m, 6H).
Mass: 525 [M+1].

### Example 20

### Preparation of (S)-N-propyl-N-[2-(4-hydroxyphenyl)ethyl]-N'-propyl-N'-[2-(5,6-dihydroxy-1,2,3,4-tetrahydro)naphthyl]-1,6-hexandiamine dihydrobromide (Compound 18)

A solution of (S)-N-propyl-N-[2-(4-methoxyphenyl)ethyl]-N'-propyl-N'-[2-(5,6-dimethoxy-1,2,3,4-tetrahydro)naphthyl]-1,6-hexandiamine (1 g; 1.9 mmoles), prepared as described in example 19, in 48% hydrobromic acid (10 ml) was heated under reflux under nitrogen for 3 hours.

The reaction mixture was then brought to dryness under reduced pressure and absolute ethanol (20 ml) was added to the residue. The solvent was evaporated, ethyl acetate (20 ml) was added and the solvent was evaporated again under reduced pressure obtaining Compound 18 (1.3 g) as an amorphous solid.

¹H-NMR (200 MHz, D₂O): δ (ppm): 0.77 (t, 3H); 0.8] (t,3H); 1.12-2.18 (m, 14H); 2.37-3.24 (m, 16H); 3.41-3.58 (m, 1H); 6.49 (d, 1H); 6.61 (d,1H); 6.65-7.06 (m, 4H).
Mass: 483 [M+1].

### Example 21

### Evaluation of the affinity towards D₁ and D₂ receptors

Brains of Sprague-Dawley male rats (200-250 g) were removed and the membranes of striated tissues were prepared according to the method described by Billard et al. in Life Sciences, 35, 1885, (1984).

The tissue were homogenized in 50 mM Tris/HCl buffer at pH 7.4 (1:100 w/v).

The homogenate was centrifuged and the pellet resuspended, recentrifuged and resuspended again in 50 mM Tris/HCl buffer at pH 7.4 containing 120 mM NaCl, 5mM KCl, 2 mM CaCl₂ and 1 mM MgCl₂.

The affinity towards D, receptor and D₂ receptor was evaluated by using [³H]-SCH23390 [R(+)-8-chloro-2,3,4,5-tetrahydro-3-methyl-5-phenyl-1H-3-benzazepine 7-ol hydrochloride] and [³H]-domperidone (The Merck Index - XI ed., no. 3412, page 537) respectively as labelled ligands.

Dopamine and dopexamine were used as reference substances.

The conditions of standard incubation (volume 1000 µl) for the test in which [³H]-SCH23390 was used were the following: 50 mM Tris/HCl buffer (pH.7.4), 0.2 nM [³H]-SCH23390, a membrane preparation of 130-140 µg proteins/ml.

The mixture was incubated with different concentrations of the tested compounds at 37°C for 20 minutes, filtered under vacuum through Whatman GF/C filters and then washed 4 times with 5 ml of 50 mM Tris/HCl buffer (pH 7.4) cooled with ice.

For the affinity studies towards D₂ receptor, [³H]-domperidone (0.3 nM) was incubated in a volume of 1000 µl containing buffer and membrane preparation as above described.

Furthermore, bovine serum albumine (BSA) (0.01%) was added.

The mixture was incubated at 37°C for 30 minutes for each concentration of tested compounds.

The obtained results, expressed as K₁ (nM), for some representative compounds of formula I, dopamine and dopexamine were reported in the following table.

**Table 1**

| Affinity [K₁ (nM)] of compounds 1-5, 7-8, 10-11, 13, 15-18, dopamine and dopexamine towards D₁ and D₂ receptors determined by binding studies on rat striated membranes. | | |
|---|---|---|
| | D₁ [³H]-SCH23390 | D₂ [³H]-domperidone |
| Compound 1 | 21 | 0.5 |
| Compound 2 | 10 | 0.2 |
| Compound 3 | 21 | 0.5 |
| Compound 4 | 33 | 0.1 |
| Compound 5 | 69 | 0.3 |
| Compound 7 | 27 | 0.5 |
| Compound 8 | 361 | 1.0 |
| Compound 10 | 40 | 0.1 |
| Compound 11 | 89 | 0.5 |
| Compound 13 | 43 | 0.8 |
| Compound 15 | 33 | 0.4 |
| Compound 18 | 107 | 0.9 |
| Dopamine | 3200 | 1500 |
| Dopexamine | 3200 | 1220 |

The compounds object of the present invention show a high affinity towards both receptor subtypes resulting more affine than dopamine and dopexamine on both D₁ and D₂ receptors.

### Example 22

### Dopaminergic functional studies on isolated tissues

### Evaluation of D₁-like activity in the Rabbit Splenic Artery (RSA) test

Arterial rings were prepared according to Semeraro et al., Naunyn. Schmied. Arch. Pharmacol., 1990, 342 539.

The arterial preparations were contracted with U46619 (9,11-dideoxy-11α,9α-epoxy-methanoprostaglandin F_{2α}) at the submaximal concentration of 0.1 µM.

The tested compounds were cumulatively administered.

Dopamine and dopexamine were used as reference substances.

The agonist activity expressed as pD2 is reported in table 2.

### Evaluation of D₂-like activity in the Rabbit Ear Artery (REA) test

Arterial rings were prepared according to the method described by Steinsland et al., Science, 1978, 443 199, modified as follows.

Male New Zealand rabbits weighing 2.5-3 Kg were sacrificed by intravenous injection of an excess of pentobarbital sodium and exsanguinated. The two ears were taken away and the central ear artery was dissected into 3 mm long rings.

These preparations were set up in a 25 ml organ bath containing Krebs solution (mM/l): NaCl 118, KCl 4.7, CaCl₂ 2.5, MgSO₄ 1.2, NaHCO₃ 25, KH₂PO₄ 1.2, glucose 11.1, equilibrated with 95% O₂-5% CO₂ and maintained at 35±1°C.

Krebs solution was medicated with EDTA (10 µM) to prevent cathecolamine oxydation, desipramine (0.1 µM) and corticosterone (30 µM), to block neuronal and extraneuronal cathecolamine uptake.

The preparations were electrically stimulated (10 Hz, 1 msec, 40-80 mA, 500 msec duration) at 5 minutes intervals.

The tested compounds were cumulatively administered.

Dopamine and dopexamine were used as reference substances.

The agonist activity expressed as pD2 is reported in table 2.

**Table 2**

| D₁-like and D₂-like activity of compounds 1-4, 6-15, 17-18, dopamine and dopexamine determined by RSA and REA tests respectively, expressed as pD2. | | |
|---|---|---|
| | D₁-like (RSA) pD2 | D₂-like (REA) pD2 |
| Compound 1 | 8.1 | 8.8 |
| Compound 2 | 8.4 | 8.8 |
| Compound 3 | 8.1 | 9.5 |
| Compound 4 | 8.5 | 10.6 |
| Compound 6 | 7.5 | 9.6 |
| Compound 7 | 7.5 | 9.7 |
| Compound 8 | 6.4 | 8.2 |
| Compound 9 | n.t. | 9.1 |
| Compound 10 | 7.2 | 9.2 |
| Compound 11 | 6.2 | 8.4 |
| Compound 12 | 7.1 | 8.6 |
| Compound 13 | 7.7 | 9.4 |
| Compound 14 | 7.9 | 9.9 |
| Compound 15 | 7.3 | 9.2 |
| Compound 18 | 6.6 | 9.2 |
| Dopamine | 6.4 | 7.8 |
| Dopexamine | 6.1 | 6.3 |

The above data show that the compounds of formula I, object of the present invention, have a dopaminergic activity significantly higher than dopamine and dopexamine.

## Claims

1. A compound of formula 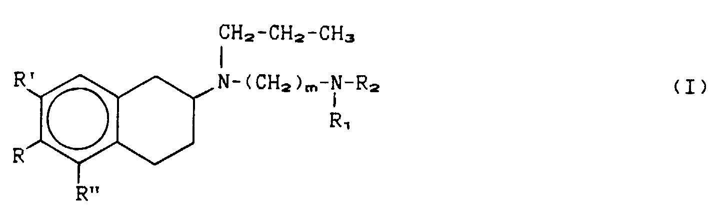 wherein
m is an integer number selected among 4, 5, 6, 7 and 8;
R, R' and R" are hydrogen atoms or OH groups, provided that at least one among R, R' and R" is a hydrogen atom but R, R' and R" are not all contemporaneously hydrogen atoms and R' and R" are not both contemporaneously OH groups;
R₁ is a hydrogen atom or a C₁-C₄ alkyl group;
R₂ is a -(CH₂)ₙ-Ar group wherein
n is an integer number selected among 2, 3, 4 and 5;
Ar is a phenyl or
R₂ is a group of formula 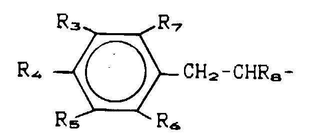 wherein one or two among R₃, R₄, R₅ and R₆ are OH groups and the remaining are hydrogen atoms;
R₇ and R₈ are hydrogen atoms or together can form a -CH₂-CH₂-chain;
and pharmaceutically acceptable salts thereof.

2. A compound according to claim 1 wherein R₂ is a group of formula -(CH₂)ₙ-Ar wherein Ar is a phenyl and n is 2 or is a group of formula 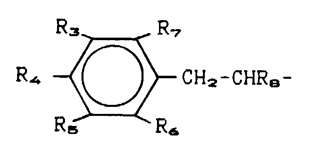 wherein one or two among R₃, R₄, R₅ and R₆ are OH groups and the remaining are hydrogen atoms; R₇ and R₈ are hydrogen atoms.

3. A compound according to claim 1 wherein m is 6; R₂ is a group of formula -(CH₂)ₙ-Ar wherein Ar is a phenyl and n is 2 or is a group of formula 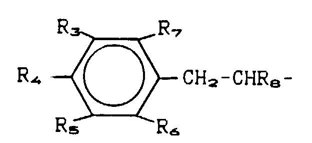 wherein one or two among R₃, R₄, R₅ and R₆ are OH groups and the remaining are hydrogen atoms; R₇ and R₈ are hydrogen atoms.

4. A compound according to claim 1 in an optically active form.

5. A process for the preparation of a compound according to claim 1 comprising the reduction of a compound of formula 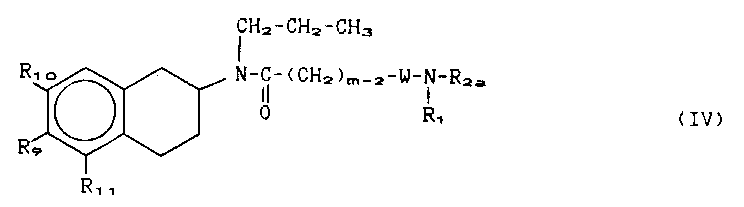 wherein m and R₁ have the meanings already reported in claim 1;
R₉, R₁₀ and R₁₁ are hydrogen atoms or OY groups wherein Y is a hydrogen atom or a protective group selected among methyl, benzyl, benzoyl and 4-methoxybenzoyl, provided that at least one among R₉, R₁₀ and R₁₁ is a hydrogen atom but R₉, R₁₀ and R₁₁ are not all contemporaneously hydrogen atoms and R₁₀ and R₁₁ are not both contemporaneously OY groups;
W is a CH₂ or CO group;
R₂ₐ has the meanings already reported in claim 1 for R₂ or is a group of formula 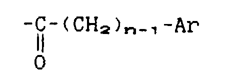 or of formula 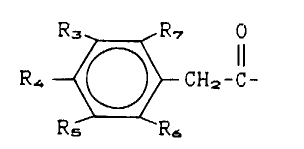 wherein n, Ar, R₃, R₄, R₅ and R₆ have the meanings already reported in claim 1; R₇ is a hydrogen atom;

6. A pharmaceutical composition containing a therapeutically effective amount of a compound according to claim 1 in admixture with a suitable carrier.

7. A pharmaceutical composition according to claim 6 for the treatment of cardiovascular diseases.

## Patentansprüche

1. Verbindung der Formel 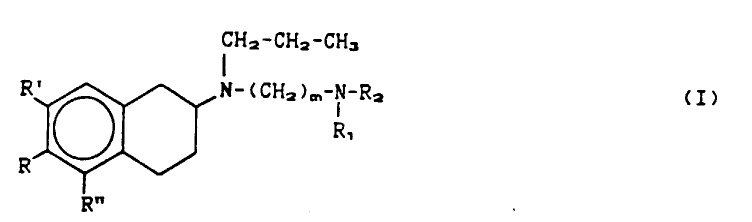 wobei
m eine aus 4, 5, 6, 7 und 8 ausgewählte ganze Zahl ist;
R, R' und R" Wasserstoffatome oder OH-Gruppen sind, vorausgesetzt, daß mindestens einer aus R, R' und R" ein Wasserstoffatom ist, aber R, R' und R" nicht alle gleichzeitig Wasserstoffatome sind und R' und R" nicht beide gleichzeitig OH-Gruppen sind;
R₁ ein Wasserstoffatom oder eine C₁-C₄-Alkylgruppe ist;
R₂ eine -(CH₂)ₙ-Ar Gruppe ist, wobei
n eine aus 2, 3, 4 und 5 ausgewählte ganze Zahl ist;
Ar ein Phenyl ist; oder
R₂ eine Gruppe der Formel 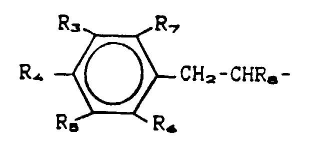 ist, wobei einer oder zwei aus R₃, R₄, R₅ und R₆ gleich OH-Gruppen sind und die verbleibenden Wasserstoffatome sind;
R₇ und R₈ Wasserstoffatome sind oder zusammen eine -CH₂-CH₂- Kette bilden'können;
und deren pharmazeutische akzeptable Salze.

2. Verbindung nach Anspruch 1, wobei R₂ eine Gruppe der Formel -(CH₂)ₙ-Ar, wobei Ar ein Phenyl ist und n gleich 2 ist, oder eine Gruppe der Formel 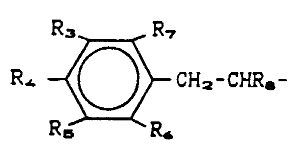 ist, wobei einer oder zwei aus R₃, R₄, R₅ und R₆ gleich OH-Gruppen sind und die verbleibenden Wasserstoffatome sind;
R₇ und R₈ Wasserstoffatome sind.

3. Verbindung nach Anspruch 1, wobei m gleich 6 ist; R₂ eine Gruppe der Formel -(CH₂)ₙ-Ar, wobei Ar ein Phenyl ist und n gleich 2 ist, oder eine Gruppe der Formel 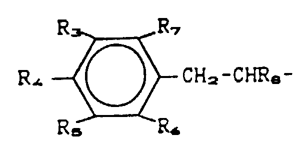 ist, wobei einer oder zwei aus R₃, R₄, R₅ und R₆ gleich OH-Gruppen sind und die verbleibenden Wasserstoffatome sind;
R₇ und R₈ Wasserstoffatome sind.

4. Verbindung nach Anspruch 1 in einer optisch aktiven Form.

5. Verfahren zur Herstellung einer Verbindung gemäß Anspruch 1, umfassend die Reduktion einer Verbindung der Formel 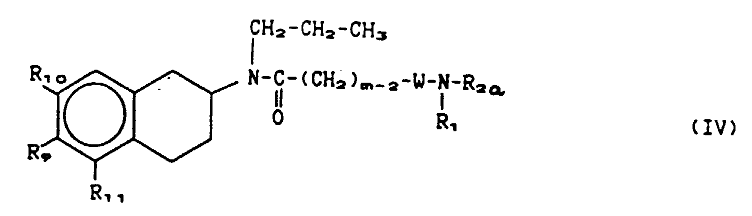 wobei m und R₁ die bereits in Anspruch 1 berichteten Bedeutungen besitzen;
R₉, R₁₀ und R₁₁ Wasserstoffatome oder OY-Gruppen sind, wobei Y ein Wasserstoffatom oder eine Schutzgruppe ist, ausgewählt aus Methyl, Benzyl, Benzoyl und 4-Methoxybenzoyl, vorausgesetzt, daß mindestens einer aus R₉, R₁₀ und R₁₁ ein Wasserstoffatom ist, aber R₉, R₁₀ und R₁₁ nicht alle gleichzeitig Wasserstoffatome sind und R₁₀ und R₁₁ nicht beide gleichzeitig OY-Gruppen sind;
W eine CH₂ oder CO-Gruppe ist;
R₂ₐ die bereits in Anspruch 1 für R₂ berichteten Bedeutungen besitzt oder eine Gruppe der Formel 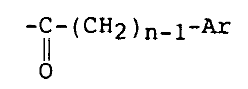 oder der Formel 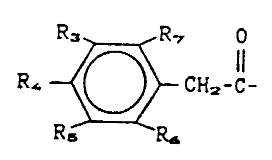 ist, wobei n, Ar, R₃, R₄, R₅ und R₆ die bereits in Anspruch 1 berichteten Bedeutungen besitzen; R₇ ein Wasserstoffatom ist.

6. Pharmazeutische Zusammensetzung, welche eine therapeutisch wirksame Menge einer Verbindung gemäß Anspruch 1 in einer Mischung mit einem geeigneten Träger besitzt.

7. Pharmazeutische Zusammensetzung nach Anspruch 6 zur Behandlung von Herz-Kreislauf-Erkrankungen.

## Revendications

1. Composé de formule : 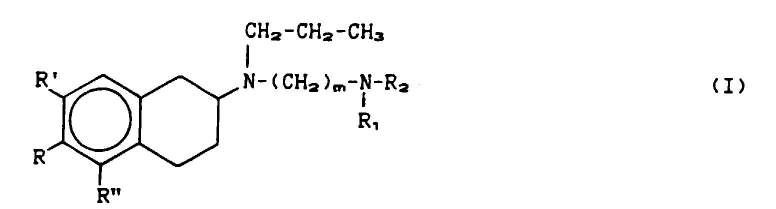 dans laquelle :
m est un nombre entier choisi parmi 4, 5, 6, 7 et 8;
R, R'et R" sont des atomes d'hydrogène ou des groupes OH, pour autant qu'au moins l'un des R, R' et R" soit un atome d'hydrogène mais que R, R' et R" ne soient pas tous simultanément des atomes d'hydrogène et que R' et R" ne soient pas tous deux simultanément des groupes OH;
R₁ est un atome d'hydrogène ou un groupe alkyle en C₁-C₄;
R₂ est un groupe -(CH₂)ₙ-Ar dans lequel :
n est un nombre entier choisi parmi 2, 3, 4 et 5;
Ar est un phényle ou bien
R₂ est un groupe de formule : 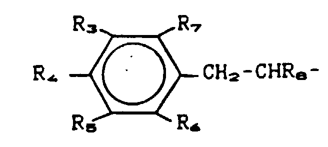 dans laquelle un ou deux des R₃, R₄, R₅ et R₆ sont des groupes OH et ceux restants sont des atomes d'hydrogène;
R₇ et R₈ sont des atomes d'hydrogène ou ensemble ils peuvent former une chaîne -CH₂-CH₂-;
et les sels pharmaceutiquement acceptables de celui-ci.

2. Composé suivant la revendication 1, dans lequel R₂ est un groupe de formule -(CH₂)ₙ-Ar dans laquelle Ar est un phényle et n est égal à 2 ou est un groupe de formule : 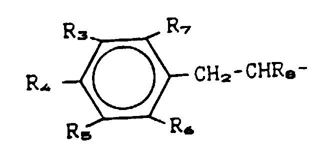 dans laquelle un ou deux des R₃, R₄, R₅ et R₆ sont des groupes OH et ceux restants sont des atomes d'hydrogène; R₇ et R₈ sont des atomes d'hydrogène.

3. Composé suivant la revendication 1, dans lequel m est égal à 6; R₂ est un groupe de formule -(CH₂)ₙ-Ar dans laquelle Ar est un phényle et n est égal à 2 ou est un groupe de formule : 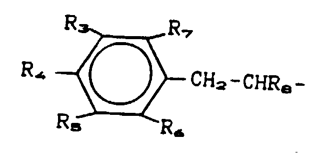 dans laquelle un ou deux des R₃, R₄, R₅ et R₆ sont des groupes OH et ceux restants sont des atomes d'hydrogène; R₇ et R₈ sont des atomes d'hydrogène.

4. Composé suivant la revendication 1, sous une forme optiquement active.

5. Procédé de préparation d'un composé suivant la revendication 1, comprenant la réduction d'un composé de formule : 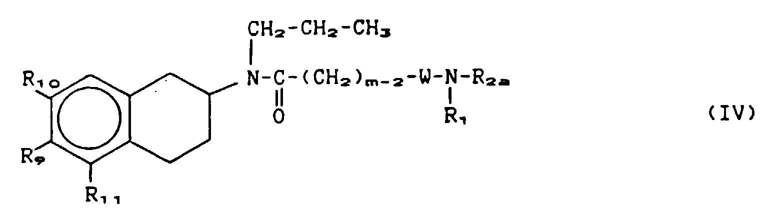 dans laquelle m et R₁ ont les significations déjà rapportées dans la revendication 1;
R₉, R₁₀ et R₁₁ sont des atomes d'hydrogène ou des groupes OY dans lesquels Y est un atome d'hydrogène ou un groupe protecteur choisi parmi les groupes méthyle, benzyle, benzoyle et 4-méthoxybenzoyle, pour autant qu'au moins l'un des R₉, R₁₀ et R₁₁ soit un atome d'hydrogène mais que R₉, R₁₀ et R₁₁ ne soient pas tous simultanément des atomes d'hydrogène et que R₁₀ et R₁₁ ne soient pas tous deux simultanément des groupes OY;
W est un groupe CH₂ ou CO;
R₂ₐ a la signification déjà rapportée dans la revendication 1 pour R₂ ou est un groupe de formule : 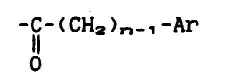 ou de formule : 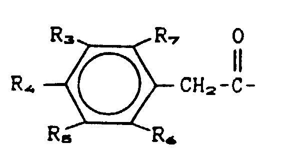 dans lesquelles n, Ar, R₃, R₄, R₅ et R₆ ont les significations déjà rapportées dans la revendication 1; R₇ est un atome d'hydrogène.

6. Composition pharmaceutique contenant une quantité thérapeutiquement efficace d'un composé suivant la revendication 1 en mélange avec un support approprié.

7. Composition pharmaceutique suivant la revendication 6 pour le traitement de maladies cardiovasculaires.
